# EUROPÄISCHE PATENTSCHRIFT

(11) **EP 3 410 143 B1**
(45) Veröffentlichungstag und Bekanntmachung des Hinweises auf die Patenterteilung: **10.07.2019**
(21) Anmeldenummer: 17173640.8
(22) Anmeldetag: 31.05.2017
(51) Int. Cl.: G01R 33/36

(54) **STECKVERBINDUNG ZUM EINSATZ IN EINEM MAGNETRESONANZGERÄT**
CONNECTOR FOR USE IN A MAGNETIC RESONANCE DEVICE
CONNECTEUR À UTILISER DANS UN APPAREIL À RÉSONANCE MAGNÉTIQUE

(43) Veröffentlichungstag der Anmeldung: 05.12.2018
(73) Patentinhaber: Siemens Healthcare GmbH, 91052 Erlangen (DE)
(72) Erfinder: Driemel, Daniel, 09569 Oederan (DE)

(56) Entgegenhaltungen:
- DE-A1-102014 109 477
- US-A- 4 516 819
- US-A1- 2005 266 708
- US-A1- 2008 044 251
- US-A1- 2013 184 563

## Beschreibung

Die Erfindung betrifft eine Steckverbindung zum Einsatz in einem Magnetresonanzgerät, ein erstes und ein zweites Verbindungsteil der Steckverbindung, eine Patientenliege und eine Lokalspule.

In der Medizintechnik zeichnet sich die Bildgebung mittels Magnetresonanz (MR), oftmals auch Magnetresonanztomographie (MRT, engl. Magnetic Resonance Imaging, MRI) genannt, durch hohe und variable Weichteilkontraste aus. Hierbei werden mit Hilfe eines Magnetresonanzgeräts Anregungspulse in einen Patienten eingestrahlt, welche im Patienten Magnetresonanzsignale auslösen. Die Magnetresonanzsignale werden durch elektrisch leitfähige Schleifen, sogenannten Spulen und/oder Antennen, empfangen. Dabei wird durch das Magnetresonanzsignal eine Spannung in der Spule induziert. Die induzierte Spannung wird üblicherweise mittels eines rauscharmen Vorverstärkers verstärkt an eine Empfangselektronik des Magnetresonanzgeräts weitergeleitet.

Die Empfangsspulen werden idealerweise möglichst nahe an dem Patienten angeordnet. Deshalb werden diese auch als Lokalspulen (engl. local coils) bezeichnet. Üblicherweise sind die Lokalspulen nicht fest mit dem Magnetresonanzgerät verbunden, sondern werden über eine Steckverbindung an das Magnetresonanzgerät angeschlossen.

Konventionelle Steckverbindungen umfassen beispielsweise geräteseitig ein erstes Verbindungsteil, beispielsweise eine Buchse an einer Patientenliege des Magnetresonanzgerätes, und spulenseitig ein zweites Verbindungsteil, beispielsweise einen Stecker an einem Kabel der Lokalspule oder auch per Direktsteckung, wie sie z.B. in den Druckschriften US 2013 0184563 A1, US 2010 0156420 A1 und US 2013 0023756 A1 offenbart wird.

Der spulenseitige Stecker umfasst oftmals Kontaktelemente, insbesondere nach außen weisende Kontaktstifte, die freiberührbar sind. Damit besteht die Gefahr einer mechanischen Beschädigung, etwa durch Verbiegen der Kontaktstifte.

Die zum Stecker korrespondierende Buchse umfasst üblicherweise Kontaktelemente, beispielsweise nach innen weisende Kontaktöffnungen, die in der Regel in einem Isolierkörper angeordnet sind. Die Kontaktelemente dürfen im Allgemeinen nicht berührbar für einen Bediener oder Patienten ausgeführt werden. Daher umfassen konventionelle Buchsen oftmals eine mechanische Abdeckung, beispielsweise mit einer gefederten Schiebe- und/oder Klappmechanik. Die Abdeckung kann vorteilhafterweise vor direkter Berührung der Kontaktelemente und/oder vor Flüssigkeitseintritt schützen. Jedoch können diese Abdeckungen auch ohne Stecker geöffnet werden, insbesondere ungewollt durch den Patienten, was zu Beschädigungen und/oder Verletzungen führen kann.

Ferner können insbesondere bei einer geöffneten Abdeckung trotzdem Flüssigkeiten in den Isolierkörper eintreten und beispielsweise zu einer Korrosion einer sich möglicherweise anschließenden Leiterplatte führen. Außerdem können nach einer Reinigung in der Buchse Flüssigkeitsrückstände von Reinigungsmitteln verbleiben, die eine Materialversprödung verursachen können, da die Reinigungsmittel in Vertiefungen der Buchse sich sammeln und über lange Zeit einwirken können.

Der Erfindung liegt die Aufgabe zugrunde, eine MR-kompatible Steckverbindung anzugeben, die komfortabel und sicher zu handhaben ist.

Die Aufgabe wird durch die Merkmale der unabhängigen Ansprüche gelöst. Vorteilhafte Ausgestaltungen sind in den Unteransprüchen beschrieben.

Demnach wird eine Steckverbindung zum Einsatz in einem Magnetresonanzgerät vorgeschlagen, die ein erstes Verbindungsteil mit zumindest einem ersten Kontaktmodul und ein zweites Verbindungsteil mit zumindest einem zweiten Kontaktmodul umfasst. Dabei sind das erste Verbindungsteil und das zweite Verbindungsteil ausgebildet, lösbar miteinander verbunden zu werden. Das erste Verbindungsteil umfasst ein Gehäuse. Das erste Verbindungsteil umfasst eine, insbesondere mechanische, Verschiebungseinheit, die ausgebildet ist, bei einem Verbinden des ersten Verbindungsteils mit dem zweiten Verbindungsteil das zumindest eine erste Kontaktmodul relativ zum Gehäuse des ersten Verbindungsteils in Richtung des zumindest einen zweiten Kontaktmoduls zu bewegen, um einen Kontakt zwischen dem zumindest einem ersten Kontaktmodul und dem zumindest einem zweiten Kontaktmodul herzustellen. Dabei ist der Kontakt vorzugsweise ein unmittelbarer und/oder direkter und/oder berührender Kontakt.

Durch einen derartigen Mechanismus kann vorteilhafterweise ein stabiler Steckzustand hergestellt werden, da durch die Relativbewegung des ersten Kontaktmoduls zum Gehäuse des ersten Verbindungsteils das erste und das zweite Kontaktmodul sicher zusammen gebracht werden können. Ferner kann vorteilhafterweise auf etwaige Schutzabdeckungen verzichtet werden.

Das erste Verbindungsteil kann beispielsweise an einer Patientenliege angeordnet sein. Das Gehäuse des ersten Verbindungsteils kann insbesondere einstückig mit der Patentenliege, insbesondere einem Gehäuse der Patientenliege, verbunden sein. Beispielsweise kann das erste Verbindungsteil in ein Gehäuse der Patientenliege integriert sein, so dass das Gehäuse der Patientenliege gleichzeitig das Gehäuse des ersten Verbindungsteils ist. Durch eine Integration des ersten Verbindungsteils in die Patientenliege können insbesondere etwaige Einzelteile und Nahtstellen zwischen solchen Einzelteilen vermieden werden. Somit kann vorteilhafterweise die Steckverbindung besonders gut gereinigt werden.

Vorzugsweise wird bei einem Verbinden des ersten Verbindungsteils mit dem zweiten Verbindungsteil das zumindest eine erste Kontaktmodul relativ zum Gehäuse des ersten Verbindungsteils in einer waagrechten Richtung bewegt. Dadurch kann möglicherweise ein ungewolltes Eindringen von Flüssigkeiten vermieden werden.

Das zweite Verbindungsteil kann beispielsweise von einem kabelgebundenen Handstecker einer Lokalspule umfasst werden, d.h. der Handstecker ist mit etwaigen Antennen der Lokalspule über ein flexibles Kabel verbunden, so dass vorteilhafterweise die Lokalspule flexibel auf einer Patientenliege positioniert werden kann.

Das zweite Verbindungsteil kann auch beispielsweise von einem kabellosen Direktstecker einer Lokalspule, beispielsweise einer Kopfspule, umfasst werden. Dabei kann insbesondere ein Gehäuse des zweiten Verbindungsteils einstückig, insbesondere starr, mit der Lokalspule verbunden sein. Beispielsweise kann das zweite Verbindungsteil in ein Gehäuse der Lokalspule integriert sein, so dass das Gehäuse der Lokalspule gleichzeitig das Gehäuse des zweiten Verbindungsteils ist. Direktsteckungen ermöglichen vorteilhafterweise einen besonders einfachen und komfortablen Arbeitsablauf.

Unter einer lösbaren Verbindung ist üblicherweise eine Verbindung gemeint, die zerstörungsfrei, insbesondere wiederkehrend zerstörungsfrei, gelöst und/oder für eine Vielzahl an Verbindungs- und/oder Trennungsvorgängen verwendet werden kann.

Unter einer mechanischen Verschiebungseinheit kann eine Einheit verstanden werden, die ausgebildet ist, das zumindest eine erste Kontaktmodul mit Hilfe von mechanischen Kräften zu bewegen. So ist etwa der Einsatz magnetischer Kräfte wegen ihrer möglichen Wechselwirkung mit etwaigen Magnetfeldern, die durch ein Magnetresonanzgerät erzeugt werden, in der Regel ungeeignet.

Das erste Verbindungsteil und das zweite Verbindungsteil können zur Herstellung einer Verbindung miteinander in Kontakt gebracht werden und/oder zur Trennung einer Verbindung voneinander entfernt werden. Der Steckverbinder ist also vorzugsweise ausgebildet, insbesondere elektrische, Leitungen zu trennen und/oder zu verbinden, über welche beispielsweise Magnetresonanzsignale übertragen werden können. Da die Steckverbindung zum Einsatz in einem Magnetresonanzgerät vorgesehen ist, umfasst die Steckverbindung vorzugsweise ausschließlich MR-kompatible, insbesondere nichtmagnetische, Materialien.

Die Steckverbindung ist vorzugsweise eine elektrische Steckverbindung, d. h. im verbundenen Zustand wird zumindest ein elektrischer Kontakt zwischen dem ersten Verbindungsteil und dem zweiten Verbindungsteil hergestellt. Über den zumindest einen Kontakt können elektrische Signale und/oder elektrische Energie übertragen werden.

Vorzugsweise weist das erste und das zweite Kontaktmodul jeweils zumindest ein elektrisch leitfähiges Kontaktelement, d.h. einen elektrischen Kontakt auf. Vorzugsweise ist in einem verbundenen Zustand das zumindest ist das elektrisch leitfähige Kontaktelement des ersten Kontaktmoduls mit dem zumindest einen elektrisch leitfähigen Kontaktelement des zweiten Kontaktmoduls, insbesondere unmittelbar, verbunden.

Unter einer relativen Bewegung des zumindest einen ersten Kontaktmoduls zum Gehäuse des ersten Verbindungsteils kann eine Bewegung verstanden werden, bei der sich die Position und/oder Lage des zumindest einen ersten Kontaktmoduls in Relation zum Gehäuse des ersten Verbindungsteils verändert. Das zumindest eine erste Kontaktmodul ist also relativ zum Gehäuse des ersten Verbindungsteils beweglich ausgeführt.

Die Steckverbindung ist derart ausgebildet, dass bei einem Verbinden des ersten Verbindungsteils mit dem zweiten Verbindungsteil das zumindest eine erste Kontaktmodul relativ zum Gehäuse des ersten Verbindungsteils in Richtung des zumindest einen zweiten Kontaktmoduls bewegt wird. Diese Verbindungsrichtung ist also bei einem Verbinden so orientiert, dass sich bei der Bewegung ein Abstand zwischen dem zumindest einen ersten Kontaktmodul und dem zumindest einen zweiten Kontaktmodul verringert.

Die Verschiebungseinheit ist ausgebildet, bei einem Trennen des ersten Verbindungsteils mit dem zweiten Verbindungsteil das zumindest eine erste Kontaktmodul relativ zum Gehäuse des ersten Verbindungsteils in eine Trennrichtung zu bewegen, die entgegengesetzt zur Verbindungsrichtung orientiert ist, um den Kontakt zwischen dem zumindest einen ersten Kontaktmodul und dem zumindest einen zweiten Kontaktmodul zu trennen.

Die Steckverbindung ist so ausgebildet, dass die Verschiebungseinheit bei einem Verbinden des ersten Verbindungsteils mit dem zweiten Verbindungsteil eine mechanische, Kraft vom zweiten Verbindungsteil auf das zumindest eine erste Kontaktmodul überträgt.

Diese übertragene Kraft wird dazu eingesetzt die Bewegung des zumindest einen ersten Kontaktmoduls relativ zum Gehäuse des ersten Verbindungsteils zu bewirken.

Bei der Steckverbindung ist vorgesehen, dass die Verschiebungseinheit des ersten Verbindungsteils eine erste Kraftübertragungseinheit umfasst und das zweite Verbindungsteil eine zweite Kraftübertragungseinheit umfasst. Dabei wird bei einem Verbinden des ersten Verbindungsteils mit dem zweiten Verbindungsteil durch die zweite Kraftübertragungseinheit eine erste, mechanische, Kraft auf die erste Kraftübertragungseinheit eingeleitet wird, so dass eine zweite, mechanische, Kraft auf das erste Kontaktmodul eingeleitet wird.

Durch die erste und zweite Kraftübertragungseinheit kann insbesondere eine äußere Kraft von außerhalb des ersten Verbindungsteils auf die Verschiebungseinheit eingeleitet werden, um die Bewegung des zumindest einen ersten Kontaktmoduls relativ zum Gehäuse des ersten Verbindungsteils zu unterstützen, insbesondere zu bewirken. Insbesondere kann durch eine externe Bewegung des zweiten Verbindungsteils die erste Kraft auf die erste Kraftübertragungseinheit eingeleitet werden, so dass die Bewegung des zumindest einen ersten Kontaktmoduls relativ zum Gehäuse des ersten Verbindungsteils von außen, insbesondere von außerhalb dem Gehäuse des ersten Verbindungsteils, beeinflusst werden kann.

Vorteilhafterweise sind die erste und die zweite Kraftübertragungseinheit so aneinander angepasst, dass die Relativbewegung des zumindest einen ersten Kontaktmoduls nur durch die zweite Kraftübertragungseinheit ausgelöst werden kann. Damit kann vorteilhafterweise eine ungewollte Betätigung des zumindest einen ersten Kontaktmoduls verhindert werden.

Eine bevorzugte Ausführungsform der Steckverbindung sieht vor, dass das Gehäuse des ersten Verbindungsteils zumindest eine Aussparung aufweist. Dabei umfasst die zweite Kraftübertragungseinheit zumindest ein vorstehendes Element, das in der zumindest einen Aussparung des Gehäuses anordbar, insbesondere einführbar, ist.

Insbesondere ist das zumindest eine vorstehende Element und die zumindest eine Aussparung zueinander korrespondierend ausgebildet. Beispielsweise ist die Form und/oder Größe des zumindest einen vorstehenden Elements und der zumindest einen Aussparung zueinander korrespondierend ausgebildet, so dass insbesondere das zumindest eine vorstehende Element in die zumindest eine Aussparung passt. Vorteilhafterweise ist die Aussparung so dimensioniert, dass keine ungewollte Betätigung der ersten Kraftübertragungseinheit, z.B. durch einen Finger eines Patienten, erfolgen kann.

Vorzugsweise kann bei einem Verbinden des ersten Verbindungsteils mit dem zweiten Verbindungsteil das zumindest eine vorstehende Element in die zumindest eine Aussparung eingeführt werden. Durch diese Einführbewegung kann insbesondere über das zumindest vorstehende Element die erste Kraft auf die erste Kraftübertragungseinheit eingeleitet werden, z.B. indem das zumindest vorstehende Element die erste Kraftübertragungseinheit wegdrückt.

Dadurch kann insbesondere eine kontrollierte, insbesondere geführte, Krafteinleitung ermöglicht werden.

Vorzugswese umfasst die die Verschiebungseinheit zumindest einen Umlenkhebel, d.h. einen drehbar gelagerten Hebel, der die erste Kraft auf zweite Kraft umlenkt. Mittels eines solchen Hebels kann vorteilhafterweise einfach und effizient eine Kraftumlenkung realisiert werden.

Vorzugsweise ist der zumindest eine Hebel innerhalb des Gehäuses des ersten Verbindungsteils angeordnet. Vorzugsweise ist der zumindest eine Hebel in einem Drehpunkt gelagert, der den zumindest einen Hebel in eine erste Seite und eine zweite Seite unterteilt. Vorzugsweise ist die erste Seite des zumindest einen Hebels an der ersten Kraftübertragungseinheit angeordnet. Vorzugsweise ist die zweite Seite des zumindest einen Hebels am zumindest einen ersten Kontaktmodul angeordnet.

Durch die Gestaltung des zumindest einen Hebels und/oder des Drehpunktes können verschiedene Übersetzungsverhältnisse zwischen einem Weg der ersten Kraftübertragungseinheit und einem Weg des ersten Kontaktmoduls realisiert werden.

Vorzugsweise weist die Verschiebungseinheit für jedes erste Kontaktmodul jeweils einen Hebel auf. Dadurch kann die Relativbewegung besonders zuverlässig durchgeführt werden.

Vorzugsweise umfasst die zweite Kraft eine Komponente, die entgegengesetzt zur ersten Kraft ausgerichtet ist. Insbesondere umfasst das erste Verbindungsteil eine Führung für das zumindest eine erste Kontaktmodul, welche unter Einwirkung der zweiten Kraft auf das zumindest eine erste Kontaktmodul eine Bewegung des zumindest einen ersten Kontaktmodul bewirkt, die entgegengesetzt zur ersten Kraft ausgerichtet ist. Vorzugsweise können etwaige Komponenten der zweiten Kraft, die nicht entgegengesetzt zur ersten Kraft ausgerichtet sind, durch diese Führung aufgenommen werden.

Vorzugsweise umfasst die Verschiebungseinheit zumindest eine Federeinheit, die der ersten Kraft entgegenwirkt. Dadurch kann in einem unverbundenen Zustand der Steckverbindung die erste Kraftübertragungseinheit in einem vorgespannten Ausgangszustand gehalten werden. Die zumindest eine Federeinheit kann beispielsweise zumindest eine, insbesondere zylinderförmige, Druckfeder umfassen.

Vorzugsweise umfasst das zweite Verbindungsteil zumindest ein Rastelement, das das zweite Verbindungsteil in einem verbundenen Zustand hält. Insbesondere kann das Rastelement einer etwaigen Federkraft der zumindest einen Federeinheit entgegenwirken.

Eine bevorzugte Ausführungsform der Steckverbindung sieht vor, dass das zumindest eine erste Kontaktmodul in einem unverbundenen Zustand gegenüber einer angrenzenden äußeren Fläche des Gehäuses des ersten Verbindungsteils bündig und/oder zurückversetzt ist. Dadurch kann vorteilhafterweise das erste Kontaktmodul gegen Beschädigungen und/oder Verschmutzungen geschützt werden.

Bei einem Verbinden des ersten Verbindungsteils mit dem zweiten Verbindungsteil wird vorzugsweise das zumindest eine erste Kontaktmodul über die besagte angrenzende äußere Fläche des Gehäuses des ersten Verbindungsteils hinausbewegt, so dass es in einem verbundenen Zustand über diese Fläche hinausragt.

Eine bevorzugte Ausführungsform der Steckverbindung sieht vor, dass das zumindest eine erste Kontaktmodul elektrisch leitfähige Kontaktelemente umfasst, die sich in einem unverbundenen Zustand innerhalb des Gehäuses des ersten Verbindungsteils befinden. Eine ungewollte Berührung der elektrisch leitfähigen Kontaktelemente kann somit vermieden werden.

Durch eine solche Anordnung können insbesondere die Kontaktelemente vor Beschädigungen und/oder Verschmutzungen geschützt werden, da sie keine äußere Angriffsfläche bieten.

Bevorzugt umfasst das zumindest eine erste Kontaktmodul elektrisch leitfähige Kontaktelemente, die in zumindest einer, insbesondere zwei parallelen, Ebenen flächig ausgebildet sind, wobei die Ebene im Wesentlichen parallel zu der Richtung orientiert ist, in der sich das zumindest eine erste Kontaktmodul bei einem Verbinden des ersten Verbindungsteils mit dem zweiten Verbindungsteil bewegt.

Vorzugsweise sind die Kontaktelemente nicht an einer Stirnseite des zumindest einen ersten Kontaktmoduls angeordnet, die in einem unverbundenen Zustand nach außen sichtbar sind, sondern beispielsweise in einer dazu senkrechten ausgerichteten Ebene.

Dies stellt eine besonders vorteilhafte Anordnung dar, um die Kontaktelemente in einem unverbundenen Zustand vor Beschädigungen und/oder Verschmutzungen zu schützen.

Bevorzugt umfasst das zweite Verbindungsteil ein Gehäuse mit zumindest einer Aussparung, wobei das zumindest eine erste Kontaktmodul bei einem Verbinden des ersten Verbindungsteils mit dem zweiten Verbindungsteil in die zumindest eine Aussparung des Gehäuses des zweiten Verbindungsteils eingeführt wird.

Vorteilhafterweise bewegt sich bei einem Verbinden des ersten Verbindungsteils mit dem zweiten Verbindungsteil das zumindest eine erste Kontaktmodul in die zumindest eine Aussparung des Gehäuses des zweiten Verbindungsteils, um einen Kontakt zwischen dem zumindest einen ersten Kontaktmodul und dem zumindest einem zweiten Kontaktmodul herzustellen.

Das zumindest eine zweite Kontaktmodul ist vorzugsweise innerhalb der zumindest einen Aussparung des Gehäuses des zweiten Verbindungsteils angeordnet. Durch diese innenliegende Anordnung kann vorteilhafterweise das zweite Kontaktmodul besser vor Schmutz und/oder Beschädigungen geschützt werden.

Vorzugsweise umfasst das zumindest eine zweite Kontaktmodul zumindest ein elektrisch leitfähiges Kontaktelement, das gefedert ist. Durch eine Federung können diese Kontaktelemente mit den Kontaktelementen des ersten Kontaktmoduls besonders zuverlässig kontaktiert werden. vorteilhafterweise können damit Fertigungstoleranzen ausgeglichen werden und/oder gleitende Kontaktierungsvorgänge umgesetzt werden.

Eine bevorzugte Ausführungsform der Steckverbindung sieht vor, dass das zweite Verbindungsteil bei einem Verbinden des ersten Verbindungsteils mit dem zweiten Verbindungsteil formschlüssig geführt wird. Eine solche Zwangsführung kann insbesondere dadurch gewährleitet werden, indem das Gehäuse des Verbindungsteils eine Form, insbesondere eine V-Form und/oder eine Nut und/oder eine Feder, aufweist, das mit einem Gegenstück einen Formschluss aufweist.

Vorteilhafterweise kann durch eine solche Zwangsführung ein komfortables und sicheres, insbesondere verkantungsfreies, Verbinden bzw. Trennen der Steckverbindung erreicht werden.

Ferner wird ein erstes Verbindungsteil vorgeschlagen, welches ausgebildet ist, mit einem zweiten Verbindungsteil eine Steckverbindung einzugehen. Ferner wird ein zweites Verbindungsteil vorgeschlagen, welches ausgebildet ist, mit einem ersten Verbindungsteil eine Steckverbindung einzugehen.

Ferner wird eine Patientenliege mit zumindest einem ersten Verbindungsteil vorschlagen. Ferner wird eine Lokalspule mit zumindest einem zweiten Verbindungsteil vorgeschlagen.

Vorzugsweise ist dabei das zumindest eine erste Verbindungsteil so auf der Patientenliege angeordnet, dass bei einem Verbinden des ersten Verbindungsteils mit einem zweiten Verbindungsteil das zumindest eine erste Kontaktmodul relativ zum Gehäuse des ersten Verbindungsteils in einer Richtung parallel zu einer Liegefläche der Patientenliege, d.h. üblicherweise einer waagrechten Richtung, bewegt wird. Dadurch kann möglicherweise ein ungewolltes Eindringen von Flüssigkeiten vermieden werden.

Bezüglich weiterer Details über das erste und zweite Verbindungsteil sowie über die Patientenliege und die Lokalspule wird auf die vorangehenden Ausführungen verwiesen.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: ein Magnetresonanzgerät mit einer Patientenliege, einer Lokalspule und einer Steckverbindung zwischen der Lokalspule und der Patientenliege in einer schematischen Darstellung,
- Fig. 2: eine Steckverbindung in einem unverbundenen Zustand in einer schematischen Darstellung,
- Fig. 3: eine Steckverbindung in einem verbundenen Zustand in einer schematischen Darstellung,
- Fig. 4: ein zweites Verbindungsteil und eine Patientenliege mit drei ersten Verbindungsteilen in einer Detaildarstellung,
- Fig. 5: ein zweites Verbindungsteil in einer Detaildarstellung,
- Fig. 6: eine Innenansicht eines ersten Verbindungsteils.

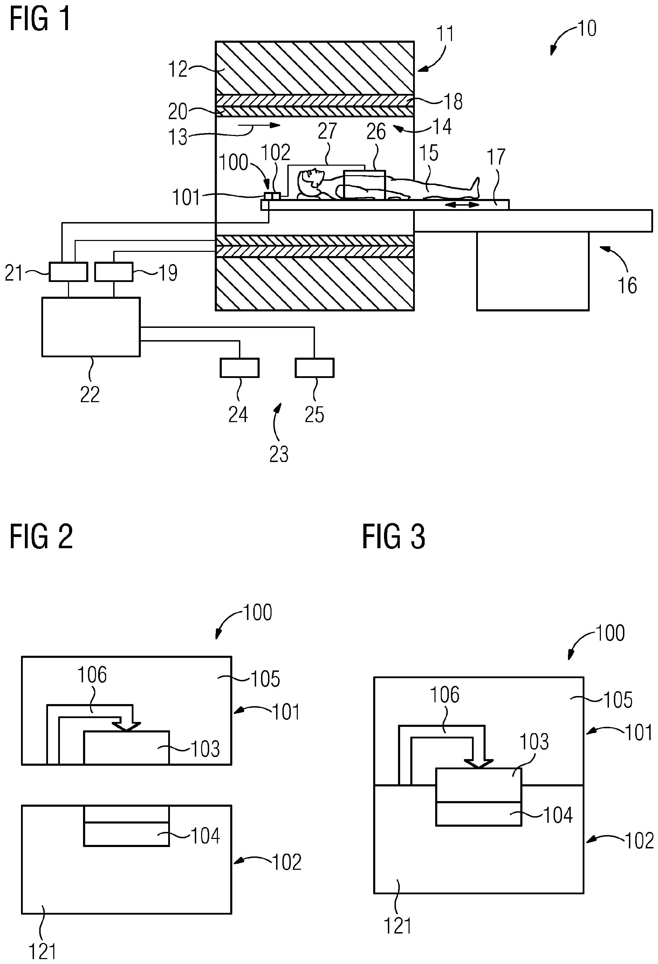

In Fig. 1 ist ein Magnetresonanzgerät 10 schematisch dargestellt. Das Magnetresonanzgerät 10 umfasst eine Magneteinheit 11, die einen Hauptmagneten 12 zu einem Erzeugen eines starken und insbesondere zeitlich konstanten Hauptmagnetfelds 13 aufweist. Zudem umfasst das Magnetresonanzgerät 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 des Magnetresonanzgeräts 10 in den Patientenaufnahmebereich 14 geschoben werden. Die Patientenlagerungsvorrichtung 16 weist hierzu eine innerhalb des Patientenaufnahmebereichs 14 bewegbar ausgestaltete Patientenliege 17 auf. Der Patient ist hierbei auf einer Liegefläche der Patientenliege 17 gelagert.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 des Magnetresonanzgeräts 10 gesteuert. Die Magneteinheit 11 umfasst weiterhin eine Hochfrequenzantenneneinheit 20, welche im vorliegenden Ausführungsbeispiel als fest in das Magnetresonanzgerät 10 integrierte Körperspule ausgebildet ist. Die Hochfrequenzantenneneinheit 20 ist zu einer Anregung von Atomkernen, die sich in dem von dem Hauptmagneten 12 erzeugten Hauptmagnetfeld 13 einstellt, ausgelegt. Die Hochfrequenzantenneneinheit 20 wird von einer Hochfrequenzantennensteuereinheit 21 des Magnetresonanzgeräts 10 gesteuert und strahlt hochfrequente Magnetresonanzsequenzen in einen Untersuchungsraum ein, der im Wesentlichen von einem Patientenaufnahmebereich 14 des Magnetresonanzgeräts 10 gebildet ist.

Die Patientenliege 17 umfasst ein erstes Verbindungsteil 101. Ferner umfasst das Magnetresonanzgerät 10 eine Lokalspule 26 mit einem Kabel 27 und einem zweiten Verbindungsteil 102, das hier als Handstecker ausgeführt ist. Das erste Verbindungsteil 101 und das zweite Verbindungsteil 102 gehen miteinander eine Steckverbindung 100 ein. Die Lokalspule 26 ist ausgebildet, Magnetresonanzsignale zu empfangen, die über die Steckverbindung 100 zur weiteren Verarbeitung übertragen werden können.

Zu einer Steuerung des Hauptmagneten 12, der Gradientensteuereinheit 19 und zur Steuerung der Hochfrequenzantennensteuereinheit 21 weist das Magnetresonanzgerät 10 eine Systemsteuereinheit 22 auf. Die Systemsteuereinheit 22 steuert zentral das Magnetresonanzgerät 10, wie beispielsweise das Durchführen einer vorbestimmten bildgebenden Magnetresonanzsequenz. Zudem umfasst die Systemsteuereinheit 22 eine nicht näher dargestellte Auswerteeinheit zu einer Auswertung der Magnetresonanzsignale, die während der Magnetresonanzuntersuchung erfasst werden. Des Weiteren umfasst das Magnetresonanzgerät 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Anzeigeeinheit 24, beispielsweise auf zumindest einem Monitor, der Benutzerschnittstelle 23 für ein Bedienpersonal angezeigt werden. Weiterhin weist die Benutzerschnittstelle 23 eine Eingabeeinheit 25 auf, mittels der Informationen und/oder Parameter während eines Messvorgangs von dem Bedienpersonal eingegeben werden können.

Fig. 2 und 3 zeigen beispielhaft jeweils eine Steckverbindung 100 zum Einsatz in einem Magnetresonanzgerät 10 in verschiedenen Zuständen, nämlich Fig. 2 in einem getrennten Zustand und Fig. 3 in einem verbundenen Zustand. Die Steckverbindung 100 umfasst ein Verbindungsteil 101 mit einem ersten Kontaktmodul 103 und ein zweites Verbindungsteil 102 mit einem zweiten Kontaktmodul 104, wobei das erste Verbindungsteil 101 und das zweite Verbindungsteil 102 ausgebildet sind, lösbar miteinander verbunden zu werden. Das zweite Verbindungsteil 102 umfasst ein Gehäuse 121. Ferner umfasst das erste Verbindungsteil ein Gehäuse 105 und eine Verschiebungseinheit 106. Die Verschiebungseinheit 106 ist ausgebildet, bei einem Verbinden des ersten Verbindungsteils 101 mit dem zweiten Verbindungsteil 102 das erste Kontaktmodul 103 relativ zum Gehäuse 105 des ersten Verbindungsteils 101 in Richtung des zweiten Kontaktmoduls 104 zu bewegen, um einen Kontakt zwischen dem ersten Kontaktmodul 103 und dem zweiten Kontaktmodul 104 herzustellen. Die weiteren Figuren zeigen Ausführungsführungsbeispiele einer solchen Steckverbindung 100.

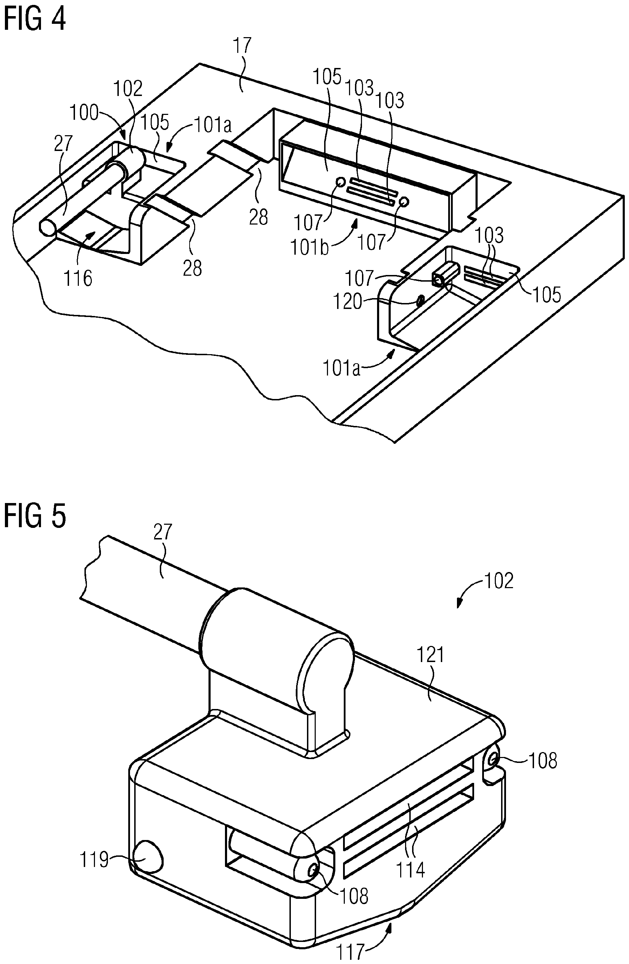

Fig. 4 zeigt eine Detaildarstellung eines Abschnitts der Patientenliege 17. Die Patientenliege 17 umfasst in diesem Beispiel drei erste Verbindungsteile 100a, 100b. Die Verbindungsteile 100a, 100b bilden Steckplätze zur Kontaktierung einer oder mehrerer Lokalspulen 26. Die beiden ersten Verbindungsteile 101a sind ausgebildet, jeweils eine Steckverbindung 100 mit einem zweiten Verbindungsteil 102 in Form eines Handsteckers einzugehen. Handstecker eigenen sich besonders, um in verschiedenen Positionen auf der Patientenliege 17 einsetzbare Lokalspulen zu kontaktieren, wie z.B. eine Body-Spule und/oder eine Flex-Spule. Solche Lokalspulen können über ein Kabel 27 mit dem zweiten Verbindungsteil 102 verbunden werden.

Ein weiteres erstes Verbindungsteil 100b ist dagegen ausgebildet, eine Steckverbindung mit einem zweiten Verbindungsteil in Form eines Direktsteckers einzugehen. Direktstecker eigenen sich besonders, um ortsfeste Lokalspulen zu kontaktieren, wie z.B. eine Kopfspule, die hier über Führungsschienen 28 eingesteckt werden können.

Die ersten Verbindungsteile 100a, 100b umfassen jeweils zwei erste Kontaktmodule 103, wobei in dieser Ansicht die zwei ersten Kontaktmodule der links dargestellten Steckverbindung 100 durch das zweite Verbindungsteil 102 verdeckt sind.

Das Gehäuse 105 der ersten Verbindungsteils Verbindungsteile 100a, 100b umfassen jeweils zwei Aussparungen, in denen erste Kraftübertragungseinheiten 107, hier in Form von Druckstangen, als Teil einer Verschiebungseinheit angeordnet sind. Die zweiten Verbindungsteile 102 umfassen dazu korrespondierende zweite Kraftübertragungseinheiten 108, mit vorstehenden Elementen, wie sie beispielhaft in Fig. 5 in Form von Führungsstiften dargestellt sind. Die vorstehenden Elemente sind in den Aussparungen anordbar, so dass beim Verbinden der Verschiebungseinheiten zweite Kraftübertragungseinheiten 108 die ersten Kraftübertragungseinheiten 107 bewegen.

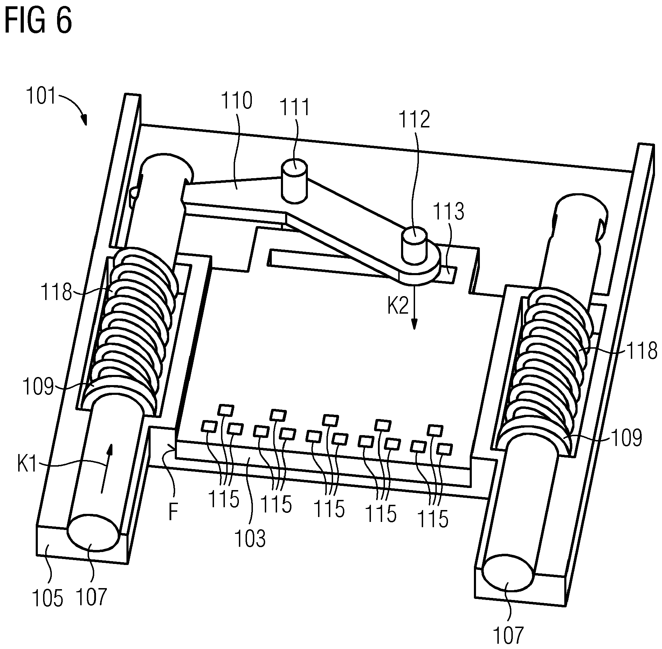

Zur Bewegung der Kontaktmodule 103 drücken die zweiten Kraftübertragungseinheiten 108, des zweiten Verbindungsteils 102, hier des Handsteckers, die ersten Kraftübertragungseinheiten 107, in die Patientenliege 17. Dabei wird eine, insbesondere mechanische, Kraft vom zweiten Verbindungsteil 102 auf die ersten Kontaktmodule übertragen. Insbesondere wird bei einem Verbinden des ersten Verbindungsteils 101 mit dem zweiten Verbindungsteil 102 durch die erste Kraftübertragungseinheit eine erste, insbesondere mechanische, Kraft K1 auf die zweite Kraftübertragungseinheit eingeleitet wird, so dass eine zweite, insbesondere mechanische, Kraft K2 auf das erste Kontaktmodul 103 eingeleitet wird, wie im Folgenden anhand von Fig. 6 erläutert wird.

Die Verschiebungseinheit umfasst eine Federeinheit 118, hier eine Druckfeder, die der ersten Kraft K1 entgegenwirkt. Die erste Kraftübertragungseinheit 107 betätigt die Federeinheit 118, die eine Rückbewegung des Kontaktmoduls 103 nach Lösen der Kontaktierung realisiert, aber nur soweit bis ein Anschlag 109 der ersten Kraftübertragungseinheit 107 das Gehäuse 105 des ersten Verbindungsteils 101 berührt. Damit kann insbesondere ein zu weites Einfahren des ersten Kontaktmoduls verhindert werden.

Die Bewegung der ersten Kraftübertragungseinheit 107 wird über einen Hebel 110 umgelenkt, der in die erste Kraftübertragungseinheit 107 eintaucht und mittels einer Achse drehbar gelagert ist, d.h. der drehbar gelagerte Hebel lenkt die erste Kraft K1 auf die zweite Kraft K2 um. Dabei ist die zweite Kraft K2 gegengesetzt zur ersten Kraft K1 ausgerichtet.

Über einen Mitnehmer 112, der mit einem Langloch 113 im ersten Kontaktmodul 103 ein Schubgelenk bildet, wird das erste Kontaktmodul 103 aus einer Parkposition in der Patientenliege 17 in eine Aussparung 114, wie beispielhaft in Fig. 5 als Schlitz dargestellt, eines Gehäuses 121 des zweiten Verbindungsteils 102 eingeschoben, d.h. das erste Kontaktmodul 103 wird bei einem Verbinden des ersten Verbindungsteils 101 mit dem zweiten Verbindungsteil 102 in die Aussparung 114 des Gehäuses 121 des zweiten Verbindungsteils 102 eingeführt. Vorteilhafterweise ist das erste Kontaktmodul 103 im Gehäuse 105 durch eine Linearführung klemmfrei geführt.

Das erste Kontaktmodul 103 ist einem unverbundenen Zustand, wie in Fig. 6 dargestellt, gegenüber einer angrenzenden äußeren Fläche F des Gehäuses 105 des ersten Verbindungsteils 101 bündig oder (leicht) zurückversetzt. Das erste Kontaktmodul umfasst elektrisch leitfähige Kontaktelemente 115, die sich in einem unverbundenen Zustand innerhalb des Gehäuses 105 des ersten Verbindungsteils 101 befinden.

Die elektrisch leitfähigen Kontaktelemente 115 sind in einer Ebene E flächig ausgebildet, wobei die Ebene E im Wesentlichen parallel zu der Richtung orientiert ist, in der sich das erste Kontaktmodul 103 bei einem Verbinden des ersten Verbindungsteils 101 mit dem zweiten Verbindungsteil 102 bewegt.

Das zweite Kontaktmodul 104 im Gehäuse 121 umfasst ebenfalls elektrisch leitfähige Kontaktelemente, die vorzugsweise in der Aussparung 114 angeordnet sind. Die flächigen Kontaktelemente 115 werden bei einem Verbinden des ersten Verbindungsteils 101 mit dem zweiten Verbindungsteil 102 über die elektrisch leitfähigen Kontaktelemente des zweiten Kontaktmoduls 104 elektrisch kontaktiert. Die elektrisch leitfähigen Kontaktelemente des zweiten Kontaktmoduls 104 können insbesondere gefedert ausgebildet sein, z.B. als biegsame Kontaktfedern. Die elektrischen leitfähigen Kontaktelemente 115 können einseitig und/oder mehrseitig, insbesondere auf zwei parallelen Ebenen, und/oder allseitig auf dem ersten Kontaktmodul 103 angeordnet sein.

Die in Fig. 4 beschriebene Ausführungsform der Steckverbindung umfasst je Steckplatz zwei erste Kontaktmodule 103. Es sind jedoch auch nur ein erstes Kontaktmodule 103 oder mehr als zwei erste Kontaktmodule 103 denkbar. Ebenso können die einzelnen Steckplätze unterschiedlich bestückt sein. Die Form der Kontaktmodule 103 ist im Ausführungsbeispiel flach gestaltet, sie kann aber auch jede andere sinnvolle Form aufweisen. Beispielsweise könnte das erste Kontaktmodul 103 als Leiterplatte ausgebildet sein, auf der die elektrischen leitfähigen Kontaktelemente 115 und optional Leiterbahnen zur Signalweiterleitung vorhanden sind. Dadurch kann ein besonders kostengünstiges erstes Kontaktmodul 103 hergestellt werden. Zur Übergabe von Signalen vom ersten Kontaktmodul 103 zur Patientenliege 17 können am ersten Kontaktmodul 103 zum Beispiel elektrische Leitungen direkt angelötet und/oder angesteckt werden.

Wie beispielhaft in Fig. 4 und 5 dargestellt, weist das zweite Verbindungsteil 102 bodenseitig eine V-förmige Gestaltung 117 auf. Die Patientenliege 17 weist an den Steckplätzen eine passende Gegenform 116 auf. Dadurch wird ein Formschluss erzielt, der eine bessere Führung des ersten Verbindungsteils 102 gewährleistet. Der Formschluss kann geometrisch auch anderes gestaltet sein, z.B. rund, als Nut und Feder etc. Im Moment des Eintauchens der zweiten Kraftübertragungseinheit 108 in die Aussparung des Gehäuses 105 des ersten Verbindungsteils 101 wird eine Zwangsführung erzeugt, so dass das zweite Verbindungsteil nicht mehr verkanten kann und das erste Kontaktmodul 103 in die korrespondierende Aussparung 114 des Gehäuses des zweiten Verbindungsteils 102 eintaucht.

Da die Kontaktierung gegen die Kraft der Federeinheit 118 erfolgt, wird vorteilhafterweise verhindert, dass die zweite Verbindungseinheit 102 wieder zurückgedrückt wird. Dies sichern im zweiten Verbindungsteils 102, insbesondere federn gelagerte, Rastelemente 119, die in formschlüssige Vertiefungen 120 in der Patientenliege 17 eintauchen. Alternativ ist es auch denkbar, dass sich entsprechende Vertiefungen des zweiten Verbindungsteils 102 befinden, in welche Rastelemente eintauchen können, die an der Patientenliege angeordnet sind. Damit kann das zweite Verbindungsteil 102 in einem verbundenen Zustand gehalten werden. Die Haltekraft ist vorteilhafterweise so dimensioniert, dass sie größer ist als eine Auswerfkraft der Federeinheit 118. Im Falle einer Direktsteckung kann diese Anforderung z.B. durch die Gewichtskraft einer Kopfspule erfüllt werden. Üblicherweise werden die Steckkraft und die Kraft beim Ziehen des zweiten Verbindungsteils bei einem Handstecker durch das Bedienpersonal aufgebracht.

Es wird abschließend noch einmal darauf hingewiesen, dass es sich bei den vorhergehend detailliert beschriebenen Verfahren sowie bei der dargestellten Erfassungsmustererzeugungseinheit und Magnetresonanzvorrichtung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

## Patentansprüche

1. Steckverbindung (100) zum Einsatz in einem Magnetresonanzgerät (10),
wobei die Steckverbindung (100) ein erstes Verbindungsteil (101) mit zumindest einem ersten Kontaktmodul (103) und ein zweites Verbindungsteil (102) mit zumindest einem zweiten Kontaktmodul umfasst,
wobei das erste Verbindungsteil (101) und das zweite Verbindungsteil (102) ausgebildet sind, lösbar miteinander verbunden zu werden,
wobei das erste Verbindungsteil (101) ein Gehäuse umfasst, wobei das erste Verbindungsteil (101) eine Verschiebungseinheit (106) umfasst, die ausgebildet ist, bei einem Verbinden des ersten Verbindungsteils (101) mit dem zweiten Verbindungsteil (102) das zumindest eine erste Kontaktmodul (103) relativ zum Gehäuse (105) des ersten Verbindungsteils (101) in Richtung des zumindest einen zweiten Kontaktmoduls (104) zu bewegen, um einen Kontakt zwischen dem zumindest einen ersten Kontaktmodul (103) und dem zumindest einen zweiten Kontaktmodul (104) herzustellen,
wobei die Verschiebungseinheit (106) ausgebildet ist, bei einem Verbinden des ersten Verbindungsteils (101) mit dem zweiten Verbindungsteil (102) eine Kraft (K1) vom zweiten Verbindungsteil (102) auf das zumindest eine erste Kontaktmodul (103) zu übertragen,
wobei die übertragene Kraft (K2) dazu eingesetzt wird, die Bewegung des zumindest einen ersten Kontaktmoduls (101) relativ zum Gehäuse (105) des ersten Verbindungsteils (101) zu bewirken.

2. Steckverbindung (100) nach einem der vorangehenden Ansprüche,
wobei die Verschiebungseinheit (106) des ersten Verbindungsteils (101) eine erste Kraftübertragungseinheit (107) umfasst und das zweite Verbindungsteil (102) eine zweite Kraftübertragungseinheit umfasst,
wobei bei einem Verbinden des ersten Verbindungsteils (101) mit dem zweiten Verbindungsteil (102) durch die zweite Kraftübertragungseinheit eine erste, insbesondere mechanische, Kraft auf die erste Kraftübertragungseinheit (107) eingeleitet wird, so dass eine zweite, insbesondere mechanische, Kraft auf das zumindest eine erste Kontaktmodul (103) eingeleitet wird.

3. Steckverbindung (100) nach Anspruch 2,
wobei das Gehäuse (105) des ersten Verbindungsteils (101) zumindest eine Aussparung aufweist,
wobei die zweite Kraftübertragungseinheit (107) zumindest ein vorstehendes Element umfasst, das in der zumindest einen Aussparung des Gehäuses (105) anordbar ist.

4. Steckverbindung (100) nach einem der Ansprüche 2 oder 3, wobei die Verschiebungseinheit (106) zumindest einen drehbar gelagerten Hebel umfasst, der die erste Kraft auf zweite Kraft umlenkt.

5. Steckverbindung (100) nach einem der Ansprüche 2 bis 4, wobei umfasst die zweite Kraft eine Komponente, die entgegengesetzt zur ersten Kraft ausgerichtet ist.

6. Steckverbindung (100) nach einem der Ansprüche 2 bis 5, wobei die Verschiebungseinheit (106) zumindest eine Federeinheit umfasst, die der ersten Kraft entgegenwirkt.

7. Steckverbindung (100) nach einem der vorangehenden Ansprüche,
wobei das zweite Verbindungsteil (102) zumindest ein Rastelement umfasst, das das zweite Verbindungsteil (102) in einem verbundenen Zustand hält.

8. Steckverbindung (100) nach einem der vorangehenden Ansprüche,
wobei das zumindest eine erste Kontaktmodul (103) in einem unverbundenen Zustand gegenüber einer angrenzenden äußeren Fläche des Gehäuses (105) des ersten Verbindungsteils (101) bündig und/oder zurückversetzt ist.

9. Steckverbindung nach einem der vorangehenden Ansprüche, wobei das zumindest eine erste Kontaktmodul (103) elektrisch leitfähige Kontaktelemente (115) umfasst, die sich in einem unverbundenen Zustand innerhalb des Gehäuses des ersten Verbindungsteils (101) befinden.

10. Steckverbindung (100) nach einem der vorangehenden Ansprüche,
wobei das zumindest eine erste Kontaktmodul (103) elektrisch leitfähige Kontaktelemente (115) umfasst, die in zumindest einer Ebene flächig ausgebildet sind, wobei die zumindest eine Ebene im Wesentlichen parallel zu der Richtung orientiert ist, in der sich das zumindest eine erste Kontaktmodul (103) bei einem Verbinden des ersten Verbindungsteils (101) mit dem zweiten Verbindungsteil (102) bewegt.

11. Steckverbindung (100) nach einem der vorhergehenden Ansprüche,
wobei das zweite Verbindungsteil (102) ein Gehäuse mit zumindest einer Aussparung (114)
umfasst,
wobei das zumindest eine erste Kontaktmodul (103) bei einem Verbinden des ersten Verbindungsteils (101) mit dem zweiten Verbindungsteil (102) in die zumindest eine Aussparung (114) des Gehäuses des zweiten Verbindungsteils (102) eingeführt wird.

12. Steckverbindung (100) nach einem der vorangehenden Ansprüche,
wobei das zumindest eine zweite Kontaktmodul elektrisch leitfähige Kontaktelemente umfasst, die gefedert sind.

13. Steckverbindung (100) nach einem der vorangehenden Ansprüche,
wobei das zweite Verbindungsteil (102) bei einem Verbinden des ersten Verbindungsteils (101) mit dem zweiten Verbindungsteil (102) formschlüssig geführt wird.

14. Erstes Verbindungsteil (101), welches ausgebildet ist, mit einem zweiten Verbindungsteil (102) eine Steckverbindung (100) nach einem der vorhergehenden Ansprüche einzugehen.

15. Zweites Verbindungsteil (102), welches ausgebildet ist, mit einem ersten Verbindungsteil (101) eine Steckverbindung (100) nach einem der Ansprüche 1 bis 13 einzugehen.

16. Patientenliege (17) mit zumindest einem ersten Verbindungsteil (101) nach Anspruch 14.

17. Lokalspule (26) mit zumindest einem zweiten Verbindungsteil (102) nach Anspruch 15.

## Claims

1. Plug connection (100) for use in a magnetic resonance device, wherein the plug connection (100) comprises a first connecting part (101) with at least one first contact module (103) and a second connecting part (102) with at least one second contact module,
wherein the first connecting part (101) and the second connecting part (102) are embodied to be connected detachably to one another,
wherein the first connecting part (101) comprises a housing, wherein the first connecting part (101) comprises a displacement unit (106), which is embodied, during a connection of the first connecting part (101) to the second connecting part (102), to move the at least one first contact module (103) relative to the housing (105) of the first connecting part (101) in the direction of the at least one second contact module (104), in order to establish contact between the at least one first contact module (103) and the at least one second contact module (104),
wherein the displacement unit (106) is embodied, during a connection of the first connecting part (101) to the second connecting part (102), to transmit a force (K1) from the second connecting part (102) to the at least one first contact module (103),
wherein the transmitted force (K2) is used to bring about the movement of the at least one first contact module (101) relative to the housing (105) of the first connecting part (101).

2. Plug connection (100) according to one of the preceding claims,
wherein the displacement unit (106) of the first connecting part (101) comprises a first force transmission unit (107) and the second connecting part (102) comprises a second force transmission unit,
wherein during a connection of the first connecting part (101) to the second connecting part (102), a first force, in particular a mechanical force, is introduced by the second force transmission unit into the first force transmission unit (107), so that a second force, in particular a mechanical force is introduced into the at least one first contact module (103).

3. Plug connection (100) according to claim 2,
wherein the housing (105) of the first connecting part (101) comprises at least one recess,
wherein the second force transmission unit (107) comprises at least one projecting element, which is able to be arranged in the at least one recess of the housing (105).

4. Plug connection (100) according to one of claims 2 or 3,
wherein the displacement unit (106) comprises at least one pivotably supported lever, which redirects the first force to the second force.

5. Plug connection (100) according to one of claims 2 to 4,
wherein the second force comprises a component that is aligned in opposition to the first force.

6. Plug connection (100) according to one of claims 2 to 5,
wherein the displacement unit (106) comprises at least one spring unit, which counteracts the first force.

7. Plug connection (100) according to one of the preceding claims,
wherein the second connecting part (102) comprises at least one latching element, which holds the second connecting part (102) in a connected state.

8. Plug connection (100) according to one of the preceding claims,
wherein the at least one first contact module (103), in an unconnected state, is flush and/or set back in relation to an adjoining outer surface of the housing (105) of the first connecting part (101).

9. Plug connection according to one of the preceding claims, wherein the at least one first contact module (103) comprises electrically-conductive contact elements (115), which in an unconnected state, are located within the housing of the first connecting part (101).

10. Plug connection (100) according to one of the preceding claims,
wherein the at least one first contact module (103) comprises electrically-conductive contact elements (115), which in at least one plane are embodied flat, wherein the at least one plane is essentially oriented in parallel to the direction in which the at least one first contact module (103) moves during a connection of the first connecting part (101) to the second connecting part (102) .

11. Plug connection (100) according to one of the preceding claims,
wherein the second connecting part (102) comprises a housing with at least one recess (114),
wherein the at least one first contact module (103), during a connection of the first connecting part (101) to the second connecting part (102), is inserted into the at least one recess (114) of the housing of the second connecting part (102).

12. Plug connection (100) according to one of the preceding claims,
wherein the at least one second contact module comprises electrically-conductive contact elements that are spring-loaded.

13. Plug connection (100) according to one of the preceding claims,
wherein the second connecting part (102), during a connection of the first connecting part (101) to the second connecting part (102), is guided to make a form fit.

14. First connecting part (101), which is embodied to enter into a plug connection (100) with a second connecting part (102) according to one of the preceding claims.

15. Second connecting part (102), which is embodied to enter into a plug connection (100) with a first connecting part (101) according to one of claims 1 to 13.

16. Patient couch (17) with at least one first connecting part (101) according to claim 14.

17. Local coil (26) with at least one second connecting part (102) according to claim 15.

## Revendications

1. Connexion (100) par fiche à utiliser dans un appareil (10) à résonnance magnétique,
la connexion (100) par fiche comprenant une première partie (101) de connexion, ayant au moins un premier module (103) de contact, et une deuxième partie (102) de connexion, ayant au moins un deuxième module de contact,
dans laquelle la première partie (101) de connexion et la deuxième partie (102) de connexion sont constituées pour être reliées l'une à l'autre de manière amovible,
dans laquelle la première partie (101) de connexion comprend un boîtier, la première partie (101) de connexion comprenant une unité (106) de déplacement, constituée pour, lors d'une liaison de la première partie (101) de connexion à la deuxième partie (102) de connexion, déplacer le au moins un premier module (103) de contact par rapport au boîtier (105) de la première partie (101) de connexion, en direction du au moins un deuxième module (104) de contact, afin de produire un contact entre le au moins un premier module (103) de contact et le au moins un deuxième module (104) de contact,
l'unité (106) de déplacement étant constituée pour appliquer, lors d'une liaison de la première partie (101) de connexion à la deuxième partie (102) de connexion, une force (K1) de la deuxième partie (102) de connexion au au moins un premier module (103) de contact,
la force (K2) appliquée étant réglée de manière à provoquer le déplacement du au moins un premier module (101) de contact par rapport au boîtier (105) de la première partie (101) de connexion.

2. Connexion (100) par fiche suivant l'une des revendications précédentes,
dans laquelle l'unité (106) de déplacement de la première partie (101) de connexion comprend une première unité (107) d'application d'une force et la deuxième partie (102) de connexion comprend une deuxième partie d'application d'une force,
dans laquelle, lors d'une liaison de la première partie (101) de connexion à la deuxième partie (102) de connexion, il est appliqué, par la deuxième unité d'application d'une force, une première force, notamment mécanique, à la première unité (107) d'application d'une force, de manière à appliquer une deuxième force, notamment mécanique, au au moins un premier module (103) de contact.

3. Connexion (100) suivant la revendication 2,
dans laquelle le boîtier (15) de la première partie (101) de connexion a au moins un évidement,
dans laquelle la deuxième unité (107) d'application d'une force comprend au moins un élément en saillie, qui peut être mis dans le au moins un évidement du boîtier (105).

4. Connexion (100) suivant l'une des revendications 2 ou 3, dans laquelle l'unité (106) de déplacement comprend au moins un levier monté tournant, qui renvoie la première force sur la deuxième force.

5. Connexion (100) suivant l'une des revendications 2 à 4, dans laquelle la deuxième force comprend une composante dirigée dans le sens contraire à la première force.

6. Connexion (100) suivant l'une des revendications 2 à 5, dans laquelle l'unité (106) de déplacement comprend au moins une unité de ressort, qui s'oppose à la première force.

7. Connexion (100) suivant l'une des revendications précédentes,
dans laquelle la deuxième partie (102) de connexion comprend au moins un élément d'encliquetage, qui maintient la deuxième partie (102) de connexion dans un état relié.

8. Connexion (100) suivant l'une des revendications précédentes,
dans laquelle le au moins un premier module (103) de contact est à affleurement et/ou en retrait à l'état non relié par rapport à une surface extérieure voisine du boîtier (105) de la première partie (101) de connexion.

9. Connexion suivant l'une des revendications précédentes, dans laquelle le au moins un premier module (103) de contact comprend des éléments (105) de contact conducteurs de l'électricité, qui, dans un état non relié, se trouvent à l'intérieur du boîtier de la première partie (101) de connexion.

10. Connexion (100) suivant l'une des revendications précédentes,
dans laquelle le au moins un premier module (103) de contact comprend des éléments (115) de contact conducteurs de l'électricité, qui sont constitués en étant en surface dans au moins un plan, le au moins un plan étant sensiblement parallèle à la direction dans laquelle le au moins un premier module (103) de contact se déplace lors d'une liaison de la première partie (101) de connexion à la deuxième partie (102) de connexion.

11. Connexion (100) suivant l'une des revendications précédentes,
dans laquelle la deuxième partie (102) de connexion comprend un boîtier ayant au moins un évidement (114),
dans laquelle le au moins un premier module (103) de contact est, lors d'une liaison de la première partie (101) de connexion et de la deuxième partie (102) de connexion, introduit dans le au moins un évidement (114) du boîtier de la deuxième partie (102) de connexion.

12. Connexion (100) suivant l'une des revendications précédentes,
dans laquelle le au moins un deuxième module de contact comprend des éléments de contact conducteurs de l'électricité, qui sont suspendus sur ressort.

13. Connexion (100) suivant l'une des revendications précédentes,
dans laquelle la deuxième partie (102) de connexion est, lors d'une liaison de la première partie (101) de connexion et la deuxième partie (102) de connexion, guidée à complémentarité de forme.

14. Premier partie (101) de connexion constituée pour entrer, avec la deuxième partie (102) de connexion, dans une liaison (100) par fiche suivant l'une des revendications précédentes.

15. Deuxième partie (102) de connexion constituée pour entrer, avec une première partie (101) de connexion, dans une liaison (100) par fiche suivant l'une des revendications 1 à 13.

16. Couchette (17) de patient, ayant au moins une première partie (101) de connexion suivant la revendication 14.

17. Bobine (26) locale, ayant au moins une deuxième partie (102) de connexion suivant la revendication 15.
